# EUROPEAN PATENT SPECIFICATION

(11) **EP 1 492 586 B1**
(45) Date of publication and mention of the grant of the patent: **21.01.2009**
(21) Application number: 03711756.1
(22) Date of filing: 04.04.2003
(51) Int. Cl.: A61M 5/30

(54) **NEEDLELESS SYRINGE FOR THE SUBCUTANEOUS INJECTION OF DROPLETS OF LIQUID SUBSTANCES**
NADELLOSE SPRITZE ZUR SUBKUTANEN INJEKTION VON TROPFEN VON FLÜSSIGEN WIRKSTOFFEN
SERINGUE SANS AIGUILLE POUR L'INJECTION SOUS-CUTANEE DE GOUTTELETTES DE SUBSTANCES LIQUIDES

(30) Priority: 05.04.2002 CA 2380671
(43) Date of publication of application: 05.01.2005
(73) Proprietor: Société de Commercialisation des Produits de la Recherche Appliquée - Socpra Sciences et Génie S.E.C., Sherbrooke, QC J1K 2R1 (CA)
(72) Inventor: BROUILLETTE, Martin, Sherbrooke, Quebec J1J 4L4 (CA); DUFRESNE, Stépfane, Saint-Félix de Valois, Québec J0K 2M0 (CA)
(74) Representative: Camus, Olivier Jean-Claude
(86) International application number: PCT/CA2003/000516
(87) International publication number: WO 2003/086510

(56) References cited:
- WO-A-00/64514
- FR-A- 1 041 887
- FR-A- 2 800 619
- FR-A- 2 804 329
- US-A- 5 954 689
- US-B1- 6 220 141

## Description

### FIELD OF THE INVENTION

The present invention relates to a needleless syringe for the subcutaneous injection of droplets of liquid substances in the skin or other target tissue of a patient, as well as a method for injection.

### BACKGROUND OF THE INVENTION

U.S. Patent No. 5,899,880 granted to Bellhouse et al. on May 4th, 1999 and U.S. Patent No. 5,865,796 (McCabe) issued on February 2, 1999 relate to devices for the high velocity injection of medicinal powders through the skin or other target tissue of a patient by means of high pressure gas.

More specifically, U.S. Patent No. 5,899,880 describes a needeless syringe capable of accelerating the medicine to speeds sufficient to obtain the desired therapeutic effect. A company, Powderject Research Limited in the United Kingdom and Powderject Vaccines, Inc. in the United States, has been founded to exploit that concept.

U.S. Patent No. 5,865,796 describes an essentially similar device, developed for the benefit of the same company. This device is destined to a usage in laboratory, for injecting genetic material.

The two above devices are designed for use with a medicine under the form of powder. They accelerate an inert gas by means of a supersonic gas flow.

The following companies already commercialize needleless syringes:
- Powderject (www.powderject.com) uses a supersonic gaseous flow to accelerate microscopic particles in order to inject these particles into the skin or other target tissue of a subject;
- Bioject (www.bioject.com) uses a micro-jet of high pressure liquid for the needleless penetration of the skin or other target tissue of a subject;
- Advantajet (www.advantajet.com) uses a micro-jet of low pressure liquid, this system being optimized for insulin;
- Mediject (www.mediject.com) uses an insulin injection system similar to that of Advantajet; and
- Many other companies exploit the concept of high pressure liquid jet for the needleless injection of liquids.

Document FR 1 041 887 describes a jet injector.

### SUMMARY OF THE INVENTION

According to the present invention, there is provided a method for needleless injection of a liquid substance as defined in claim 1.

The present invention further relates to a needless syringe according to claim 8.

Other aspects of the invention are defined in the dependant claims.

The foregoing and other objects, advantages and features of the present invention will become more apparent upon reading of the following non restrictive description of illustrative embodiments thereof, given by way of example only with reference to the accompanying drawings.

### BRIEF DESCRIPTION OF THE DRAWINGS

In the appended drawings:
Figure 1 is a schematic diagram illustrating the structure and operation of a first illustrative embodiment of needleless syringe in accordance with the present invention;
Figure 2 is a schematic diagram illustrating the structure and operation of a second illustrative embodiment of needleless syringe in accordance with the present invention;
Figure 3a is a first schematic diagram showing details of the structure and operation of the second illustrative embodiment of needleless syringe of Figure 2;
Figure 3b is a second schematic diagram showing details of the structure and operation of the second illustrative embodiment of needleless syringe of Figure 2;
Figure 3c is a third schematic diagram showing details of the structure and operation of the second illustrative embodiment of needleless syringe of Figure 2;
Figure 4 is a schematic diagram illustrating a process of production of droplets as used in the first and second illustrative embodiments of needleless syringe as shown in Figures 1 and 2.
Figure 5 is a schematic diagram illustrating the structure of a third illustrative embodiment of needleless syringe in accordance with the present invention;
Figure 6 is a schematic diagram illustrating the structure of a fourth illustrative embodiment of needleless syringe in accordance with the present invention;
Figure 7 is a schematic diagram illustrating the structure of a needleles syringe not part of the present invention;
Figure 8 is a schematic diagram illustrating the structure of another needleless syringe not part of the present invention;
Figure 9a is a first schematic diagram illustrating the structure and operation of a last needleless syringe not part of the present invention;
Figure 9b is a second schematic diagram illustrating the structure and operation of the last needleless syringe; and
Figure 9c is a third schematic diagram illustrating the structure and operation of the last needleless syringe

### DETAILED DESCRIPTION OF THE ILLUSTRATIVE EMBODIMENTS

The illustrative embodiments of the present invention will now be described with reference to the accompanying drawings.

The present invention enables the injection of liquid substances, under the form of droplets, in the skin or other target tissue of a patient without directly perforating the skin or other target tissue as this is the case when a syringe with a needle is used. The liquid substances of concern may comprise, , in particular but not exclusively, vaccines, anaesthetics, medicines, hormones, genetic compounds, etc. The small size of the droplets and the speed of these droplets as generated by the needleless syringe allow the liquid substances to penetrate the skin or other target tissue of the patient and reach a specific tissue target.

More specifically, sufficiently small droplets are generated and accelerated to a sufficiently high velocity to allow these droplets to penetrate the patient's skin or other target tissue and thereby produce the desired medical effect. The advantages of such an approach over needles are obvious; the three main advantages are:
- the increase in efficiency of the treatment, by targeting the desirable cells and tissues, more specifically but not restricted to, in the epidermis and dermis;
- the reduction of the risk of infection; and
- almost complete elimination of patients' pain and fear.

### First illustrative embodiment of the needleless syringe:

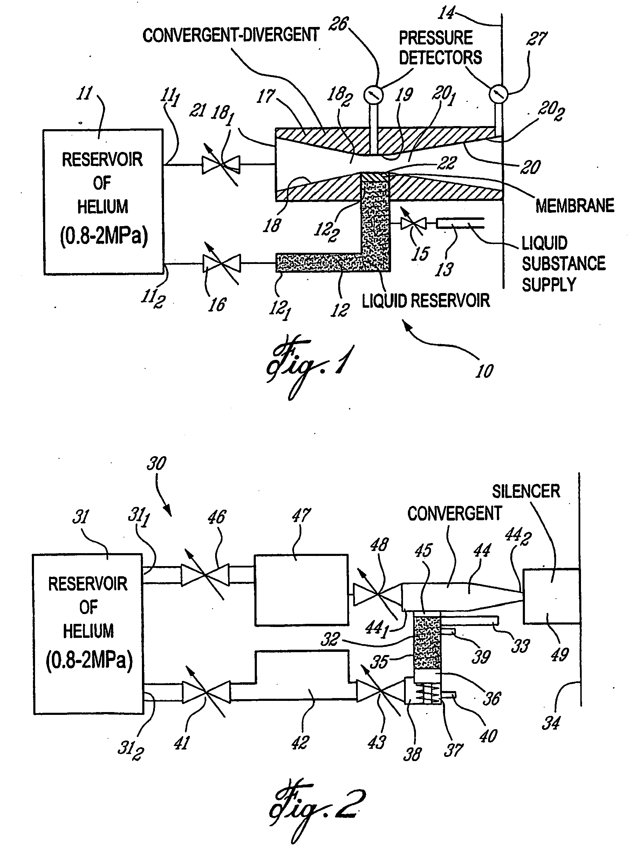

Referring to Figure 1, the first illustrative embodiment of needleless syringe 10 comprises a reservoir 11 containing pressurized gas. As a non limitative example, the pressurized gas comprises a high pressure inert gas such as helium at a pressure of 0.8 to 2 MPa.

The needleless syringe 10 also comprises a reservoir of liquid 12 in which the liquid substance to be injected in the patient's skin or other target tissue 14 is supplied from a liquid substance supply 13 through a valve 15. The quantity of liquid substance will depend, in particular but not exclusively, on the required dosage. The reservoir 12 is L-shaped and comprises a first lower free end 12₁ connected to an outlet 11₂ of the reservoir 11 of pressurized gas through a valve 16.

The needleless syringe 10 further comprises a convergent-divergent 17 comprising serially interconnected convergent 18, throat 19 and divergent 20.

The convergent 18 comprises an end 18₁ of larger diameter connected to an outlet 11₁ of the reservoir 11 of pressurized gas through a valve 21. The convergent 18 also comprises an end 18₂ of smaller diameter connected to one side of the throat 19.

The L-shaped reservoir 12 comprises a second upper end 12₂ communicating with the throat 19 through a perforated membrane 22, for example a metallic or polymeric membrane 22 formed with at least one micro-orifice.

The divergent 20 comprises an end of smaller diameter 20₁ connected to the other side of the throat 19. Finally, the divergent 20 comprises a free end 20₂ of larger diameter which is open and, in operation, applied next to the surface of the skin or other target tissue 14 of the patient directly or through a spacer/silencer (not shown in Figure 1).

The illustrative embodiment of needleless syringe 10 generally comprises two systems:
- a first system for generating droplets (generator of droplets) of given small size at a pre-selected flow rate; and
- a second system for accelerating the droplets (droplet accelerator) at a pre-selected velocity while directing these droplets toward a predetermined location of the surface of the skin or other target tissue.

### First system for generating droplets:

Referring to Figure 1 of the appended drawings, the first system for generating small droplets of given size at a pre-selected flow rate will now be described.

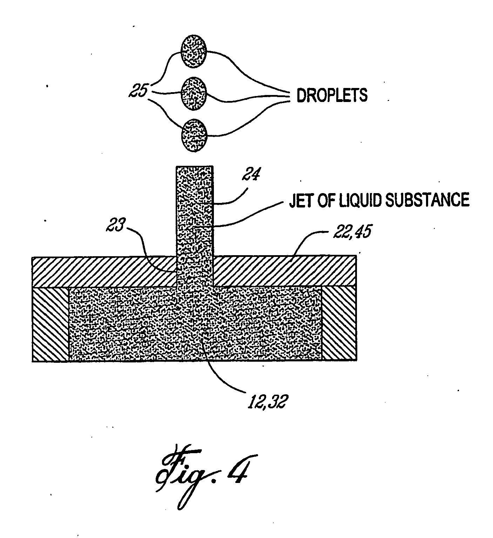

As illustrated in Figure 1, when the valve 16 is opened, gas under pressure is supplied from the outlet 11₂ of the gas reservoir 11 to the inlet 12₁ of the liquid reservoir 12. The pressure of the gas in the reservoir 12 forces the liquid substance from the reservoir 12 through the micro-orifice such as 23 (Figure 4) of the perforated membrane 22. On the side of the membrane 22 opposite to the reservoir 12, a jet 24 is produced by the liquid substance flowing through the micro-orifice 23. This jet is then transformed into droplets such as 25 via natural flow instabilities caused by the surface tension of the liquid.

Although the example of perforated membrane 22 as illustrated in Figure 4 comprises only one micro-orifice, it should be kept in mind that the number and diameter of the micro-orifices such as 23 are determined in relation to the intended application. Moreover, the rate of flow of the liquid substance through the membrane 22 will be determined in relation to the number and the size of the micro-orifces 23 through the membrane 22 as well as the applied pressure at the inlet 12₁ of the liquid reservoir 12. In this droplet generating system, the size of the droplets 25 is controlled by the diameter of the orifice 23. Usually, it is found that the droplet size is about twice the orifice size.

In the first illustrative embodiment, the system for producing the droplets 25 uses a perforated membrane 22 comprising at least one micro-orifice trough which liquid substance is forced by means of a pressure exerted upstream of the membrane 22. Another example of system for producing microscopic droplets 25 of controllable size can use an ultrasonic atomizer, such as those used in ultrasonic humidifiers.

### Second system for accelerating the droplets:

As a non-limitative example, the second system for accelerating the droplets 25 (droplet accelerator) comprises the reservoir 11 of pressurized gas, the valve 21 and the convergent-divergent 17 (Figure 1).

In operation, opening of the valve 21 will cause a high velocity jet of gas from the outlet 11₁ through the convergent 18, the throat 19 and the divergent 20, to convey the droplets 25 produced within the throat 19 and to inject these droplets 25 in the patient's skin or other target tissue 14. An advantage connected to the use of a convergent-divergent 17 is the production of an almost steady state supersonic jet of gas.

An alternative to the use of a convergent-divergent 17 to accelerate the droplets 25 is the use of a shock tube or an expansion tube for producing a generally non-stationary flow with a certain duration of steady state flow velocity.

According to another alternative, the system for accelerating the droplets 25 can use electrostatic acceleration. In that particular case, the droplets 25 are first electrically charged, for example through a frictional process. The charged droplets are then subjected to an electrostatic field created between two parallel plates. The two parallel plates can be perforated or not, and are disposed parallel to the injection target surface. Of course, potentials of opposite polarities are respectively applied to the parallel plates. A similar device would use a magnetic field or a combination of various electromagnetic fields to produce droplet acceleration.

According to a further alternative that will be described in more detail in the following description, the system for accelerating the droplets 25 can use direct injection of the liquid substance. For that purpose, a high speed jet of the liquid substance is produced upstream of a membrane formed with micro-orifices. The jet of liquid substance becomes unstable and is transformed into a train of droplets. In accordance with this alternative, the perforated membrane is positioned close to the injection target surface parallel thereto.

Accordingly, the present invention proposes different methods for producing droplets and different methods for accelerating these droplets. It will readily appear to those of ordinary skill in the art that several combinations of these different methods for producing the droplets and these different methods for accelerating these droplets can be implemented.

It should also be pointed out that, in the case of droplet accelerating systems using a droplet-conveying high velocity jet of gas, the droplets can be injected at different locations of the high velocity jet of gas, and that whatever the droplet producing method being used. For example, when a convergent-divergent 17 is used to produce the quasi steady-state high velocity jet of gas, the droplets can be introduced in this high velocity jet of gas upstream of the throat 19, downstream this throat 19, or directly within that throat 19 of the convergent-divergent 17.

### Operation of the first illustrative embodiment of needleless syringe:

The first illustrative embodiment of the needleless syringe 10 of Figure 1 operates as follows:
- the valve 21 is opened to supply gas under pressure from the outlet 11₁ of the reservoir 11 through the convergent-divergent 17 until a steady-state high velocity jet of gas is established through this convergent-divergent 17;
- the valve 16 is then opened to supply gas under pressure from the outlet 11₂ of the reservoir 11 to the liquid reservoir 12 and thereby force liquid from the reservoir 12 through the above described at least one micro-orifice of the membrane 22;
- as described with reference to Figure 4, passage of liquid substance through each micro-orifice such as 23 of the membrane 22 produces, on the side of the membrane 22 opposite to the reservoir 12, a jet such as 24 of liquid substance, this jet being transformed into droplets such as 25; and
- the droplets 25 are accelerated by the high velocity jet of gas established through the convergent-divergent 17, in particular through the throat 19, toward the patient's skin or other target tissue 14; it should be pointed out that, in the first illustrative embodiment of Figure 1, the droplets are accelerated by the viscous forces generated by a difference in velocity between the gas jet and the droplets.

Although this is not required for the needleless syringe to operate, the valve 21 is advantageously opened before the valve 16 is opened to enable the establishment of a steady-state high velocity jet of gas through the convergent-divergent 17 before the droplets 25 are produced and introduced in this high velocity jet of gas. This ensures a uniform velocity of the droplets 25.

Since the velocity and the size of the droplets control the depth of penetration of these droplets through the skin or other target tissue of the patient, it is appropriate to optimize these two parameters in order to maximize the therapeutic efficiency for a particular liquid medical formulation.

The theory related to the first illustrative embodiment of the needleless syringe as illustrated in Figure 1 has been developed and the resulting model shows that the velocity of the jet of gas produced in the convergent-divergent 17 essentially varies as a function of:
- the ratio between the pressure of the gas in the reservoir 11 and the pressure at the output, that is at the free end 20₂ of the divergent 20;
- the thermodynamic properties of the gas in the reservoir 11, in particular the speed of the sound (which depends on the temperature) and the ratio of the specific heats of this gas; and
- the ratio of the inner cross-section of the throat 19 and the inner cross-section of the output (free end 20₂) of the convergent-divergent 17;
and the velocity of the droplets 25 essentially varies as a function of:
- the size of the droplets;
- the viscosity of the gas used to convey the droplets;
- the axial length of the divergent 20; and
- the velocity profile of the gas in the convergent-divergent.

In an exemplary, non-limitative set-up of the first illustrative embodiment of the needleless syringe according to the present invention, the inner cross-section of the throat 19 is 50 mm², while the inner cross-section of the free end 20₂ of the divergent 20 is 77 mm², for an area ratio of about 1.5.

With this ratio of cross-sections, a pressure of 410 kPa in the throat 19 and an output pressure of 101.3 kPa at the free end 20₂ of the divergent 20, the output velocity of the gas at the free end 20₂ of the divergent 20 is 800 m/s. These pressure values were measured by means of two pressure sensors 26 and 27 positioned as illustrated in Figure 1.

Then, a volume of water of 1 cc was injected in the liquid reservoir 12 through the valve 15. The membrane 22 comprised six (6) micro-orifices such as 25 with an average diameter of 200 microns. To determine the size of the droplets 25, the mechanics of liquid jets was used; the theory [ANNO, J.N. (1977), The Mechanics of Liquid Jets, Lexington Book, p. 103] states that the diameter of the generated droplets are about two times larger that the diameter of the micro-orifice. Consequently, the resulting droplets 25 had a diameter of about 400 microns, which is relatively large.

In order to determine whether the droplets were susceptible to penetrate the skin or other target tissue of a patient, tests have been made on the skin or other target tissue of a subject. To better visualise the level of penetration, the droplets have also been coloured. As a result, it was observed that the droplets could penetrate the target skin or other target tissue at a depth of about 1 mm under the above conditions.

The above numerical example clearly shows that control of both the size of the droplets 25 through the diameter of the micro-orifices such as 23 of the membrane 22 and the speed of the droplets 25 through the properties of the high velocity jet of gas, the depth of penetration of the liquid substance through the subject's skin or other target tissue could be controlled with high accuracy and reliability.

### Second illustrative embodiment of the needleless syringe:

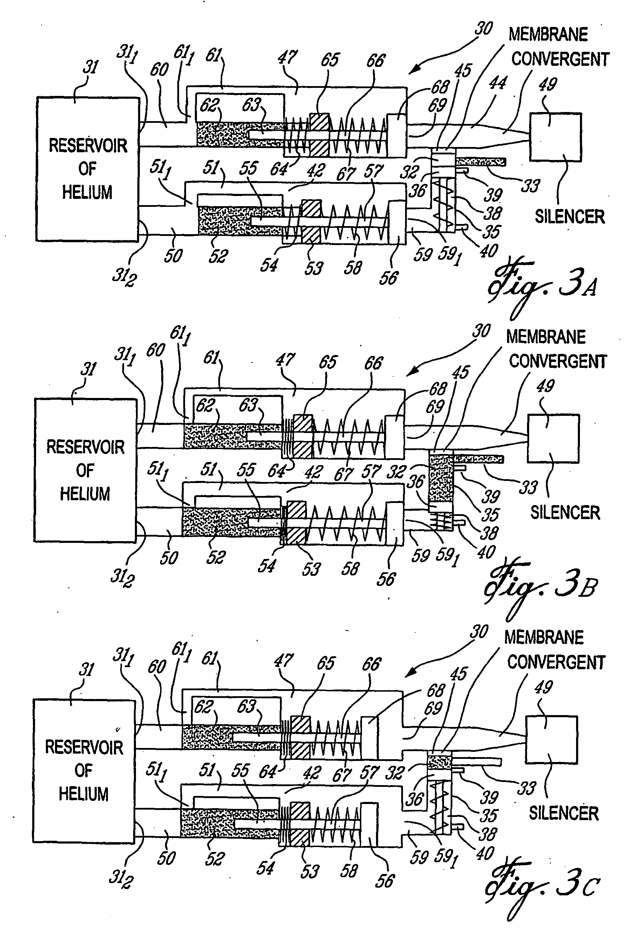

A second illustrative embodiment of needleless syringe is generally designated by the reference 30 in Figures 2, 3a, 3b, 3c and 3d.

Referring to Figure 2, the second illustrative embodiment of the needleless syringe 30 comprises a reservoir 31 containing a pressurized gas. For example, the pressurized gas comprises a high pressure inert gas such as helium at a pressure located within the range of 0.8 to 2 MPa.

The needleless syringe 30 also comprises a liquid reservoir 35 with a chamber 32 containing the liquid substance to be injected in the patient's skin or other target tissue 34. In the second illustrative embodiment of Figure 2, the liquid reservoir 35 is advantageously cylindrical to receive a spring-biased piston 36. More specifically, piston 36 is spring-biased toward the liquid chamber 32 by means of a helical spring 37 positioned in a gas-tight chamber 38. As illustrated in Figure 2, the piston 36 is slidably mounted in the liquid reservoir 35 to divide this liquid reservoir 35 into the liquid chamber 32 and gas-tight chamber 38.

The liquid substance to be injected can be introduced in the liquid reservoir 32 through an inlet port 33. The inlet port 33 presents any suitable design allowing the liquid substance to be introduced in the liquid chamber 32 but preventing this liquid substance to escape from the liquid chamber 32 after it has been introduced in this liquid chamber 32. A first purging element 39 mounted on the liquid chamber 35 and a second purging element 40 mounted on the gas-tight chamber 38 can be opened whenever required. For example, opening of at least the purging element 40 during the supply of liquid substance in the liquid chamber 39 will allow gas to escape from the chamber 38 to thereby facilitate the introduction of the liquid substance in the liquid chamber 32. The quantity of liquid substance introduced in the liquid chamber 32 will depend, in particular but not exclusively, on the desired dosage.

The gas-tight chamber 38 is connected to an outlet 31₂ of the reservoir 31 of pressurized gas through serially interconnected valve 41, intermediate chamber 42 and valve 42.

The needleless syringe 30 further comprises a convergent 44. Indeed, a convergent such as 44 can be used in the place of a convergent-divergent such as 17 of Figure 1 when the required gas velocity is lower than the speed of the sound. However, it should be kept in mind that a convergent-divergent could be used in the place of the convergent in the second illustrative embodiment as illustrated in Figure 2. As well, a convergent could be used in the place of the convergent-divergent in the first illustrative embodiment as illustrated in Figure 1.

The upper end of the liquid chamber 32 communicates with the convergent 44 through a perforated membrane 45, for example a metallic or polymeric membrane 45 formed with at least one micro-orifice. The perforated membrane 45 can be similar to the above-described perforated membrane 22 of Figure 1.

The end of larger diameter 44₁ of the convergent 44 is connected to an outlet 31₁ of the reservoir 31 of pressurized gas through serially interconnected valve 46, intermediate chamber 47 and valve 48.

The convergent 44 comprises an end of smaller diameter 44₂ applied to or next to the skin or other target tissue 34 of the patient through a silencer and/or spacer 49. The function of the silencer 49 is to damp the sound produced by the flow of fluid. The function of the spacer is to ensure a desired stand-off distance between the end 44₂ and the skin or other target tissue 34.

Again, the second illustrative embodiment of needleless syringe 30 generally comprises two systems:
- a first system for generating droplets (droplet generator) of given size at a pre-selected flow rate; and
- a second system for accelerating the droplets (droplet accelerator) at a pre-selected velocity and directing these droplets toward a predetermined location of the surface of the skin or other target tissue..

### First system for producing droplets:

Referring to Figure 2 of the appended drawings, the first system for generating droplets of given size at a pre-selected flow rate comprises:
- the reservoir of pressurized gas 31;
- the valve 41;
- the intermediate chamber 42;
- the valve 43;
- the liquid reservoir 35 including the gas-tight chamber 38, the spring-biased piston 36 and helical spring 37, and the liquid chamber 32; and
- the perforated membrane 45 and its at least one micro-orifice.

The valves 41 and 43 (Figure 2) can be commercially available electronic valves suitable for this particular application and permitting an electronic control of the parameters of operation of the needleless syringe 30, in particular but not exclusively the gas pressure in the intermediate chamber 42 and 47, and the timing of the various operations.

Alternatively, Figures 3a, 3b and 3c illustrate valves 41 and 43 formed of pistons, plungers and springs. More specifically, as illustrated in Figure 3a, 3b and 3c:
- the valve 41 comprises a tubular member 50 interconnecting the intermediate chamber 42 with the outlet 31₂ of the reservoir of pressurized gas 31, a L-shaped deviation line 51 of diameter smaller that the diameter of the tubular member 50 and extending between one side of the tubular member 50 and the secondary chamber 42, a plunger 52 slidably mounted in the tubular member 50 and normally obstructing the inlet 51₁ from the tubular member 50 to the L-shaped deviation line 51, a piston 53 mounted at one end of the plunger 52, located in the intermediate chamber 42 and having a diameter larger than the diameter of the plunger 50, and a spring 54 mounted on the plunger 52 between the piston 53 and the inner surface of the intermediate chamber 42 around the tubular member 50; and
- the valve 43 comprises, inside the intermediate chamber 42, an axial hole 55 through the piston 53 and a section of the plunger 52, a piston 56 with an axial pin 57 slidably mounted in the axial hole 55, a spring 58 mounted one the axial pin 57 between the pistons 53 and 56, and a tubular member 59 between the secondary chamber 42 and the gas-tight chamber 38, the inlet 59₁ from the intermediate chamber 42 to this tubular member 59 being normally closed by the piston 56.

Therefore, the pistons slide in each other but the amplitude of their respective strokes is limited by appropriately positioned stops.

### Second system for accelerating the droplets:

As a non-limitative example, the second system for accelerating the droplets comprises the valve 46, the intermediate chamber 47, the valve 48, the convergent 44, and the spacer/silencer 49.

The valves 46 and 48 (Figure 2) can be commercially available electronic valves suitable for this particular application and permitting an electronic control of the parameters of operation of the needleless syringe, in particular but not exclusively the gas pressure in the secondary chamber 47, and the timing of the various operations.

Alternatively, Figures 3a, 3b and 3c illustrate valves 46 and 48 formed of pistons, plungers and springs. More specifically, as illustrated in Figure 3a, 3b and 3c:
- the valve 46 comprises a tubular member 60 interconnecting the intermediate chamber 47 with the outlet 31₁ of the reservoir of pressurized gas 31, a L-shaped deviation line 61 having a diameter smaller than the diameter of the tubular member 60 and extending between one side of the tubular member 60 and the intermediate chamber 47, a plunger 62 slidably mounted in the tubular member 60 and normally obstructing the inlet 61₁ from the tubular member 50 to the L-shaped deviation line 61, a piston 65 mounted at one end of the plunger 62, located in the intermediate chamber 47 and having a diameter larger than the diameter of the plunger 62, and a helical spring 64 mounted on the plunger 62 between the piston 65 and the inner surface of the intermediate chamber 47 around the tubular member 60; and
- the valve 48 comprises, inside the intermediate chamber 47, an axial hole 63 through the piston 65 and a section of the plunger 62, a piston 68 with an axial pin 66 slidably mounted in the axial hole 63, a helical spring 67 mounted on the axial pin 66 between the pistons 65 and 68, and an outlet 69 of the intermediate chamber 47 to the convergent 44 normally closed by the piston 68.

Therefore, the pistons slide in each other but the amplitude of their respective strokes is limited by appropriately positioned stops.

### Operation of the second illustrative embodiment of needleless syringe:

An example of operation of the second illustrative embodiment of the needleless syringe 30 of Figure 2 will now be described with reference to Figures 3a, 3b and 3c.

Pistons 53 and 65 are initially in the respective positions shown in Figure 3a. In these positions of the pistons 53 and 65, the plunger 52 opens the inlet 51₁ of the deviation line 51 to allow pressurized gas from the reservoir 31 to flow toward the intermediate chamber 42 through the outlet 31₂, the tubular member 50, and the deviation line 51. In the same manner, the plunger 62 opens the inlet 61₁ of the deviation line 61 to allow pressurized gas from the reservoir 31 to flow toward the intermediate chamber 47 through the outlet 31₁, the tubular member 60, and the deviation line 61.

After the intermediate chambers 42 and 47 are full of pressurized gas from the reservoir 31, the pistons 53 and 65 are moved to the respective positions shown in Figure 3b against the force produced by the springs 54 and 64, respectively. The plunger 52 then seals the inlet 51₁ while the plunger 62 seals the inlet 61₁.

A liquid substance is then introduced in the liquid chamber 32 through, for example, the inlet port 33. The purging elements 39 and 40 can be used during this operation to provide for escape of air from the chambers 32 and 38 and thereby facilitate downward movement of the piston 36 and thereby introduction of the liquid substance within the liquid chamber 32. The purging elements 39 and 40 are then sealed.

The pistons 56 and 68 are then displaced toward the pistons 53 and 65, respectively, against the force produced by the springs 58 and 67, respectively (see Figure 3c). Displacement of the piston 56 toward the piston 53 causes sliding of the axial pin 57 in the axial hole 55 and opening of the inlet 59₁ to the tubular member 59 and gas-tight chamber 38. In the same manner, displacement of the piston 68 toward the piston 65 causes sliding of the axial pin 66 in the axial hole 63 and opening of the inlet 69 to the convergent 44.

Opening of the inlet 69 causes release of the pressurized gas from the intermediate chamber 47 to the convergent 44 to produce in the convergent 44 and silencer 49 the high velocity jet of inert gas (Figure 3c).

Opening of the inlet 59₁ causes the pressurized gas from the intermediate chamber 38 to flow toward the gas-tight chamber 38 of the liquid reservoir 35 through the tubular member 59 to apply a corresponding pressure on the piston 36 and thereby force liquid substance from the liquid chamber 32 to flow through the micro-orifices such as 23 (Figure 4) to produce the jet of liquid substance 24 (Figure 4) on the side of the membrane 45 opposite to the liquid chamber 32. This jet 24 is transformed into droplets 25 (Figures 3c and 4) of liquid substance through natural flow instabilities which are caught and conveyed by the high velocity jet of gas 70 through the convergent 44 and the silencer 49 for finally being injected in the skin or other target tissue 34 (Figure 2) of the patient.

Although this is not required for the needleless syringe to operate, the valve 48 (piston 68) is advantageously opened before the valve 43 (piston 56) is opened to enable the establishment of a steady-state high velocity jet of gas through the convergent 44 before the droplets 25 are produced and introduced into this high velocity jet of gas. This ensures a uniform velocity of the droplets 25.

The needleless syringe according to the first and second illustrative embodiments may further comprise the following non-limitative variants:
- if a convergent-divergent such as 17 (Figure 1) is used, the diameter of the outlet 31₁ of the reservoir of pressurized gas 31 has a diameter equal to or larger than the diameter of the collar section 19 of this convergent-divergent 17;
- a triggering system (not shown), for example an electronic circuit or a mechanism, for operating the valves of the needleless syringe according to the first and second illustrative embodiments is provided for; this triggering system can be a mechanism for operating the pistons 53, 56, 65 and 68 as described hereinabove with reference to Figures 3a, 3b and 3c;
- the reservoir 31 of pressurized gas can be under the form of a replaceable or refillable unit;
- the needleless syringe is a disposable syringe;
- the pressure of gas in the intermediate chambers 42 and 47 can be adjusted by means of, for example, spring-biased mechanical mano-relief valves since, to obtain a uniform velocity of the droplets during the first as well as during the last injection, the gas pressure in the intermediate chambers 42 and 47 must be the uniform from one injection to the other to thereby ensure repeatability of the parameters of injection at each use of the needleless syringe;
- the gas pressure in the reservoir 31 is sufficiently high to allow the needleless syringe to perform a given number of injections;
- the membrane 22, 45 is a replaceable unit, and the number of micro-orifices such as 25 can be adapted to the flow rate of liquid substances required by the intended application;
- the liquid substance can be introduced in the liquid chamber 32 by means of a depression produced in the air-tight chamber 38 and, when the piston 36 has reached its lower position, it is blocked until the syringe is triggered;
- many alternatives for introducing the liquid substance to be injected in the liquid reservoir can be envisaged; examples are syringes with needles, preloaded capsules especially designed for the intended application, or a capsule that can be used for many injections (for example for diabetes); and
- as indicated in the foregoing description, instead of valves formed of pistons, plungers and springs as illustrated in Figure 3a, 3b and 3c, the valves 16 and 21 (Figure 1), and the valves 41, 43, 46 and 48 (Figure 2) can be commercially available electronic valves suitable for this particular application and permitting an electronic control of the parameters of operation of the needleless syringe, in particular but not exclusively the gas pressure in the intermediate chambers such as 42 and 47, and the timing of the various operations.

### Third illustrative embodiment of the needleless syringe:

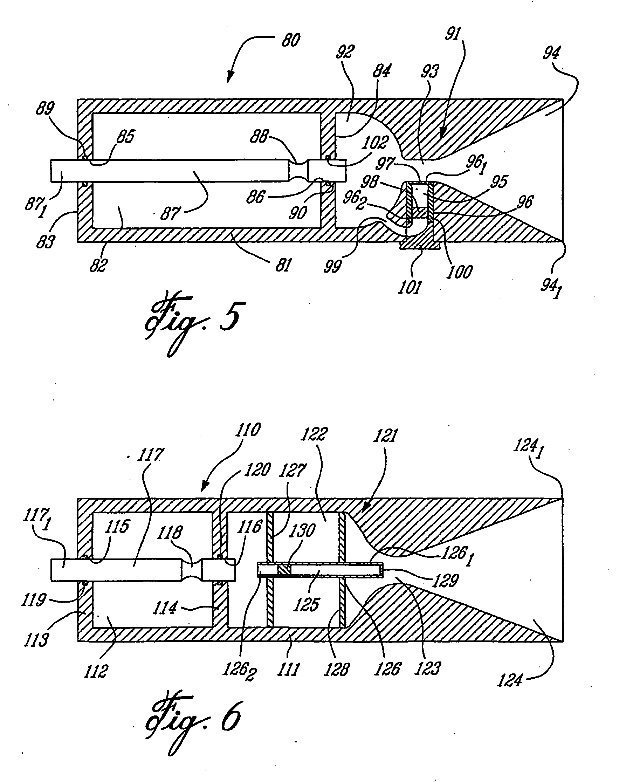

Referring to Figure 5, the third illustrative embodiment of needleless syringe 80 comprises a body 81 made, for example, of molded plastic material. The body 81 defines an axial reservoir 82 containing pressurized gas, for example a high pressure inert gas such as helium at a pressure of 0.8 to 2 MPa.

The reservoir 82 defines an end transversal wall 83 and an inner transversal wall 84. Transversal wall 83 comprises a central, axial hole 85 while transversal wall 84 comprises a central, axial hole 86. An elongated rod 87 extends through both the holes 85 and 86, this elongated rod 87 comprising an annular notch 88 inside the reservoir 82 in the proximity of the hole 86. To prevent leakage of pressurized gas from the reservoir 82, a first 0-ring 89 is mounted between the hole 85 and the outer surface of the rod 87 while a second 0-ring 102 is mounted between the hole 86 and the outer surface of the rod 87.

The body 81 of the needleless syringe 80 further comprises a convergent-divergent 91 on the side of the wall 84 opposite to the reservoir 82. The convergent-divergent 91 comprises, in series from the wall 84, a convergent 92, throat 93 and a divergent 94.

The third illustrative embodiment of needleless syringe 80 further comprises a reservoir of liquid 95 containing the liquid substance to be injected in the patient's skin or other target tissue (not shown in Figure 5). The quantity of liquid substance will depend, in particular but not exclusively, on the required dosage. In the third illustrative embodiment, the reservoir of liquid 95 is under the form of a cylinder 96 embedded in the material of the body 81, and having a first end 96₁ communicating with the throat 93 through a perforated membrane 97, for example a metallic or polymeric membrane formed with at least one micro-orifice. In the third illustrative embodiment of Figure 5, the cylinder 96 also comprises a second end 96₂ closed by means of a piston 98. The end 96₂ of the cylinder 96 communicates with the upstream end of the inside of the convergent 92 through a deviation line 99 formed in the body 81 of the third preferred embodiment of needleless syringe 80.

The third illustrative embodiment of needleless syringe 80 generally comprises two systems:
- a first system for generating droplets (generator of droplets) of given small size at a pre-selected flow rate; and
- a second system for accelerating the droplets (droplet accelerator) at a pre-selected speed while directing these droplets toward a predetermined location of the surface of the skin or other target tissue.

### First system for generating droplets:

Referring to Figure 5 of the appended drawings, the first system for generating droplets of given size at a pre-selected flow rate comprises:
- the reservoir of pressurized gas;
- the valve formed by the holes 85 and 86, the rod 87 with the annular notch 88, and the O-rings 89 and 90;
- the divergent 92 and deviation line 99; and
- the reservoir of liquid 95 comprising the cylinder 96, the perforated membrane 97 and the piston 98.

### Second system for accelerating the droplets:

As a non-limitative example, the second system for accelerating the droplets (droplet accelerator) comprises the:
- the reservoir of pressurized gas 82;
- the valve formed by the holes 85 and 86, the rod 87 with the annular notch 88, and the O-rings 89 and 90; and
- the convergent-divergent 91.

### Operation of the third illustrative embodiment of needleless syringe:

The third illustrative embodiment of needleless syringe 80 of Figure 5 operates as follows:
- the outer, free end 87₁ of the rod 87 is pushed toward the wall 83 to enables supply of pressurized gas from the reservoir 82 to the convergent 92 through the gap between the notch 88 and the hole 86 and thereby establish a high velocity jet of gas through the convergent 92, throat 93 and divergent 94 of the convergent-divergent 91;
- the pressurized gas supplied to the convergent 92 also apply a pressure to the piston 98 through the deviation line 99 to thereby force liquid substance from the reservoir 82 through the above described at least one micro-orifice of the perforated membrane 97;
- as described with reference to Figure 4, passage of liquid substance though each micro-orifice such as 23 of the perforated membrane 97 produces, on the side of the membrane 97 opposite to the reservoir 95, a jet such as 24 of liquid substance, this jet being transformed into droplets such as 25; and
- the droplets are accelerated by the high velocity jet of gas established through the convergent-divergent 91, in particular through the throat 93, toward the patient's skin or other target tissue (not shown in Figure 5).

In the third illustrative embodiment of Figure 5:
- a plug of any design can be used to cover and protect the perforated membrane 97 during storage;
- any other type of valve can be used to isolate the pressurized gas reservoir from the convergent, for example a double burstable membrane as will be described in the following description;
- a silencer (not shown) can be mounted on the free end 94₁ of the divergent 94 to reduce noise;
- a spacer (not shown) can be mounted on the free end 94₁ of the divergent 94 to ensure a preset stand-off distance from the target tissue; and/or
- the liquid reservoir 95, including the cylinder 96, the perforated membrane 97 and the piston 98 can be designed as a separate unit that can be inserted in an appropriately shaped cavity 100 of the body 81 through a side opening 101. The side opening 101 can then be sealed either permanently for example by a press-fit plug 102 or non-permanently for example through a screwed plug.

### Fourth illustrative embodiment of the needleless syringe:

Referring to Figure 6, the fourth illustrative embodiment of needleless syringe 110 also comprises a body z111 made, for example, of molded plastic material. The body 111 defines an axial reservoir 112 containing pressurized gas, for example a high pressure inert gas such as helium at a pressure of 0.8 to 2 MPa.

The reservoir 112 defines an end transversal wall 113 and an inner transversal wall 114. Transversal wall 113 comprises a central, axial hole 115 while transversal wall 114 comprises a central, axial hole 116. An elongated rod 117 extends through both the holes 115 and 116, this elongated rod 117 comprising an annular notch 118 inside the reservoir 112 in the proximity of the hole 116. To prevent leakage of pressurized gas from the reservoir 112, a first O-ring 119 is mounted between the hole 115 and the outer surface of the rod 117 while a second O-ring 120 is mounted between the hole 116 and the outer surface of the rod 117.

The body 111 of the needleless syringe 110 further comprises a convergent-divergent 121 on the side of the wall 114 opposite to the reservoir 112. The convergent-divergent 121 comprises, in series from the wall 114, a convergent 122, throat 123 and a divergent 124.

The fourth illustrative embodiment of needleless syringe 110 further comprises a reservoir of liquid 125 containing the liquid substance to be injected in the patient's skin or other target tissue (not shown in Figure 6). The quantity of liquid substance will depend, in particular but not exclusively, on the required dosage. In the fourth illustrative embodiment, the reservoir of liquid 115 is under the form of a cylinder 126 mounted axially in the convergent 122 through axially spaced apart support members 127 and 128 extending between the outer surface of the cylinder 126 and the inner surface of the convergent 122 but structured not to impede the flow of high velocity jet of gas. The cylinder 126 has a distal end 126₁ communicating with the throat 123 through a perforated membrane 129, for example a metallic or polymeric membrane formed with at least one micro-orifice. In the fourth illustrative embodiment of Figure 6, the cylinder 126 also comprises a second end 126₂ located in the proximity of the hole 116 in the wall 114 and closed by means of a piston 130.

The fourth illustrative embodiment of needleless syringe 80 generally comprises two systems:
- a first system for generating droplets (generator of droplets) of given small size at a pre-selected flow rate; and
- a second system for accelerating the droplets (droplet accelerator) at a pre-selected speed while directing these droplets toward a predetermined location of the surface of the skin or other target tissue.

### First system for generating droplets:

Referring to Figure 6 of the appended drawings, the first system for generating droplets of given size at a pre-selected flow rate comprises:
- the reservoir of pressurized gas 112;
- the valve formed by the holes 115 and 116, the rod 117 with the annular notch 118, and the O-rings 119 and 120;
- the convergent 122; and
- the reservoir of liquid 125 comprising the cylinder 126, the perforated membrane 129 and the piston 130.

### Second system for accelerating the droplets:

As a non-limitative example, the second system for accelerating the droplets (droplet accelerator) comprises the:
- the reservoir of pressurized gas 112;
- the valve formed by the holes 115 and 116, the rod 117 with the annular notch 118, and the O-rings 119 and 120; and
- the convergent-divergent 121.

### Operation of the fourth illustrative embodiment of needleless syringe:

The fourth illustrative embodiment of needleless syringe 110 of Figure 6 operates as follows:
- the outer, free end 1171 of the rod 117 is pushed toward the wall 113 to enable supply of pressurized gas from the reservoir 112 to the convergent 122 through the space between the annular notch 118 and the hole 116, and thereby establish a high velocity jet of gas through the convergent 122, throat 123 and divergent 124 of the convergent-divergent 121;
- the pressurized gas supplied to the convergent 122 also apply a pressure to the piston 130 to thereby force liquid substance from the reservoir 125 through the above described at least one micro-orifice of the perforated membrane 129;
- as described with reference to Figure 4, passage of liquid substance though each micro-orifice such as 23 of the perforated membrane 129 produces, on the side of the membrane 129 opposite to the reservoir 125, a jet such as 24 of liquid substance, this jet being transformed into droplets such as 25; and
- the droplets are accelerated by the high velocity jet of gas established through the convergent-divergent 121, in particular through the throat 123, toward the patient's skin or other target tissue (not shown in Figure 6).

In the fourth illustrative embodiment of Figure 6:
- a cap of any design can be used to cover and protect the perforated membrane 129 during storage;
- any other type of valve can be used to isolate the pressurized gas reservoir from the convergent, for example a double burstable membrane as will be describe in the following description;
- a silencer (not shown) can be mounted on the free end 124₁ of the divergent 124 to reduce noise;
- a spacer (not shown) can be mounted on the free end 124₁ of the divergent 124 to ensure a preset stand-off distance from the target tissue; and/or
- the liquid reservoir 125, including the cylinder 126, the perforated membrane 129 and the piston 130 can be designed as a separate unit that can be inserted on appropriately designed support members 127 and 128 through the end 124₁ of the divergent 124, or by separating the body 121 in the convergent region 122.

### A needleless syringe not part of the invention

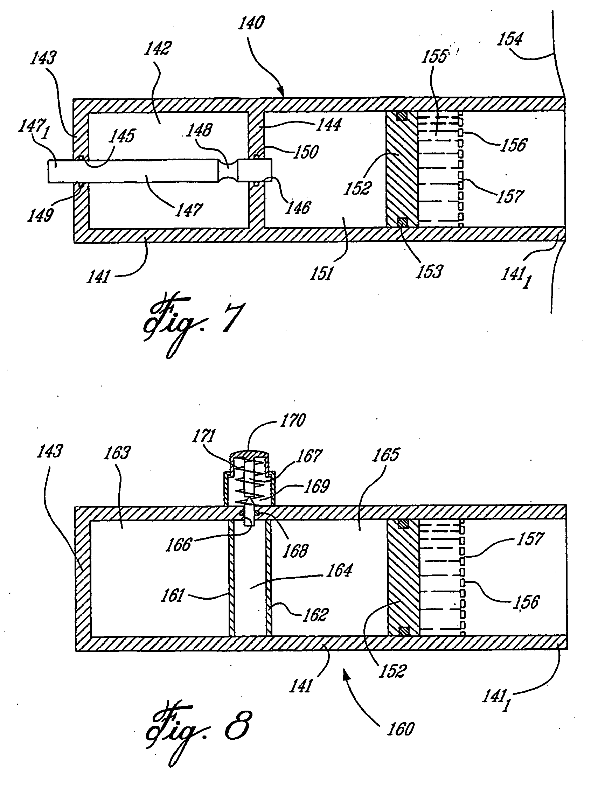

Referring to Figure 7, the needleless syringe 140 comprises a cylindrical body 141 made, for example, of molded plastic material. The body 141 defines an axial reservoir 142 containing pressurized gas, for example a high pressure inert gas such as helium at a pressure of 0.8 to 2 MPa.

The reservoir 142 defines an end transversal wall 143 and an inner transversal wall 144. Transversal wall 143 comprises a central, axial hole 145 while transversal wall 144 comprises a central, axial hole 146. An elongated rod 147 extends through both the holes 145 and 146, this elongated rod 147 comprising an annular notch 148 inside the reservoir 142 in the proximity of the hole 146. To prevent leakage of pressurized gas from the reservoir 142, a first O-ring 149 is mounted between the hole 145 and the outer surface of the rod 147 while a second O-ring 150 mounted between the hole 146 and the outer surface of the rod 147.

The body 141 of the needleless syringe 140 further defines, on the side of the wall 144 opposite to the reservoir 142 and from this wall 144 to the end 141₁ of the cylindrical body 141, a series arrangement including a chamber 151 containing gas at low pressure, for example 1 atmosphere, a piston 152 with an annular seal such as an O-ring 153 between the peripheral edge surface of the piston 152 and the inner surface of the cylindrical body 141, a chamber 155 containing liquid substance to be injected in the patient's skin or other target tissue 154, and a perforated membrane 156, for example a metallic or polymeric membrane formed with micro-orifices such as 157. Again, the quantity of liquid substance will depend, in particular but not exclusively, on the required dosage.

### Operation of the needleless syringe:

The needleless syringe 140 of Figure 7 operates as follows:
- the outer, free end 147₁ of the rod 147 is pushed toward the wall 143 to enable supply of pressurized gas from the reservoir 142 to the chamber 151 through the gap between the notch 148 and the hole 146;
- the pressurized gas supplied to the chamber 151 will apply a pressure to the piston 152 to thereby force liquid substance from the chamber 155 through the above described micro-orifices of the perforated membrane 157;
- as described with reference to Figure 4, passage of liquid substance though each micro-orifice such as 156 of the perforated membrane 156 produces, on the side of the membrane 156 opposite to the chamber 155, a jet such as 24 of liquid substance, this jet being transformed into droplets such as 25; and
- the pressure of the gas supplied in the chamber 151 is sufficiently high to project and direct the droplets of liquid substance toward the patient's skin or other target tissue with a velocity sufficient to inject these droplets of liquid substance in the patient's skin or other target tissue.

In the syringe of Figure 7:
- a plug or cap of any design can be used to cover and protect the perforated membrane 156 during storage;
- the piston 152 can be a thin flexible membrane deformed by the gas under pressure;
- a silencer (not shown) can be mounted on the free end 141₁ of the cylindrical body 141 to reduce noise;
- the above described gas driving system to apply pressure to the piston 152 or flexible membrane can be replaced by any suitable spring driver system or chemical propellant driving system; and/or
- the perforated membrane 156 is located at a pre-determined stand-off distance from the patient's skin, or other target tissue154, according to each particular application.

### Another needleless syringe not part of the invention

Referring to Figure 8, the needleless syringe 160 is similar to the syringe of Figure 7, except that the valve system of Figure 7 including the wall 144, the axial holes 145 and 146, the elongated rod 147 and the O-rings 149 and 150 is replaced by a pair of transversal, burstable membranes 161 and 162.

As illustrated in Figure 8, the reservoir of pressurized gas 163 is defined in the cylindrical body between the end wall 143 and the membrane 161, a first gas-containing chamber 164 is defined between the membranes 161 and 162, and a second gas-containing chamber 165 is defined between the membrane 162 and the piston 152. The membranes 161 and 162 are capable of withstanding a pressure of ½ p₂ or higher. As the pressure of the gas in the reservoir 163 is p₂, the pressure of the gas in the chamber 164 is about ½ p₂, and the pressure of the gas in the chamber 165 is 1 atmosphere, the membranes 161 and 162 are capable of withstanding the pressure differentials.

A hole 166 is made through the wall of the cylindrical body 141, this hole 166 opening into the chamber 164. A spring-biased plunger 167 is inserted in the hole 166 with a seal such as an O-ring 168 positioned between the plunger 167 and the hole 166 to prevent escape of gas from the chamber 164. On the outside of the cylindrical body 141, the plunger 167 is formed with an annular notch 169, and is provided with a head 170 and a spring 171 interposed between the head 170 and the outer surface of the cylindrical body 141.

Momentary depression of the spring-biased plunger 167 will allow gas from the chamber 164 to escape through the gap between the annular notch 169 and the hole 166 to thereby increase the differential of pressure between the opposite sides of the membrane 161. The pressure of the gas in the reservoir 163 will then burst the membrane 161 to supply the chamber 164 with pressurized gas which, in turn, will burst the membrane 162. Of course, the resistance of the membranes 161 and 162 are designed to burst in response to momentary depression of the plunger 167, and the plunger allows a minimal amount of gas to escape from the hole 166 but just sufficient to enable the burst of the membranes 161 and 162.

The pressurized gas from the reservoir will then apply pressure to the piston 152 with the same result as described with reference to the syringe of Figure 7.

In the syringe of Figure 8:
- a cap or plug of any design can be used to cover and protect the perforated membrane 156 during storage
- a silencer (not shown) can be mounted on the free end 141₁ of the cylindrical body 141;
- any other valve mechanism can be used to replace the hole 166, the spring-biased plunger 167, and the seal 168 in order to momentarily vent the gas from the chamber 164 such that membrane 161 is caused to burst with minimal gas from reservoir 163 escaping to the atmosphere outside the needleless syringe 160;
- the perforated membrane can be replaced by a lattice or trellis; and/or
- if in sufficiently small quantity, the liquid substance can be placed on the downstream side of the perforated membrane, lattice or trellis where it could be retained by surface tension; in this case the piston 152 would not be required.

### A last needleless syringe not part of the invention

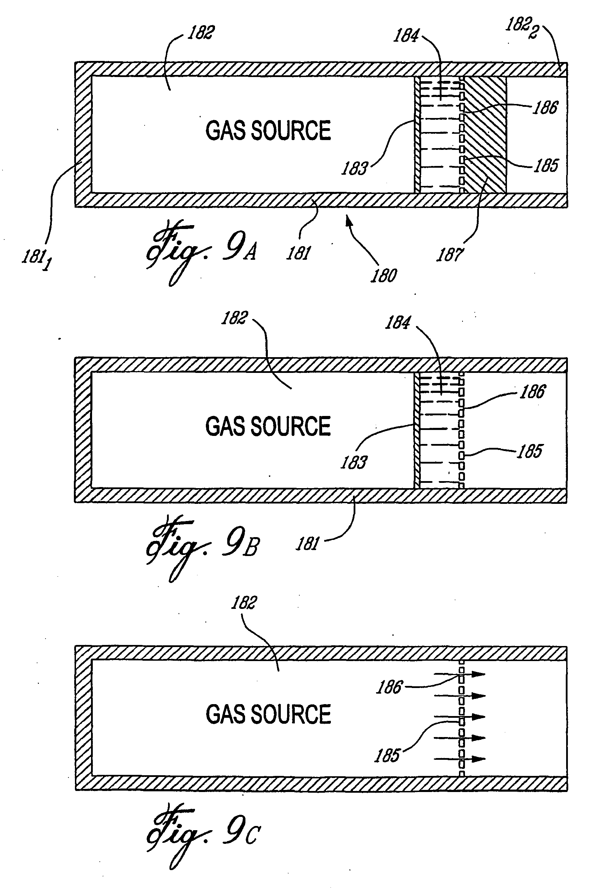

Referring to Figures 9a, 9b and 9c, the needleless syringe 180 comprises a cylindrical body 181 made, for example, of molded plastic material.

The body 181 contains, from a closed end 181₁ to an open end 181₂ thereof, a gas source 182, a burstable membrane 183, a chamber 184 containing liquid substance to be injected in the patient's skin or other target tissue, a perforated membrane185 with at least one micro-orifice 186, and a cap 187 for covering and protecting the perforated membrane 185.

For example, the gas source 182 can be formed either by:
- the reservoir 142, wall 143, wall 144, hole 145, hole 146, rod 147, O-ring 149, O-ring 150 and chamber 151 of Figure 7; or
- the burstable membranes 161 and 162, the reservoir of pressurized gas 163, the first gas-containing chamber 164, the second gas-containing chamber 165, and the spring-biased plunger 167 of Figure 8.

### Operation of the last needleless syringe not part of the invention

The needleless syringe 140 of Figure 9a, 9b and 9c operates as follows:
- the cap 187 is removed (Figure 9b);
- pressurized gas from the gas source 182 is released as described, for example, either with reference to Figures 7 or 8 to apply a pressure to the burstable membrane 183;
- the resistance of the membrane 183 is so selected that the released pressurized gas will burst this membrane 183 (Figure 9b); and
- after bursting of the membrane 183, the pressurized gas forces liquid substance through the micro-orifices 186 of the membrane 185 to produce both high velocity jets of gas and droplets accelerated to a sufficiently high velocity for being injected in the patient's skin or other target tissue.

In a general, non-limitative manner, the size of the droplets such as 25 (Figure 4) will normally vary within the range of 1 to 1000 microns and the velocity of the jet of gas within the range of 10 to 1000 m/s, depending on the specific application. Of course, the present invention is intended to cover sizes and velocities located outside of these ranges.

Although the present invention has been described hereinabove with reference to illustrative embodiments thereof, it should be kept in mind that these illustrative embodiments can be modified at will, within the scope of the appended claims, without departing from the scope of the subject invention.

## Claims

1. A method for needleless injecting a liquid substance into a target biological tissue (14, 34, 154), whereby said substance is not delivered to the human or animal living body, the method comprising:
producing a high velocity jet of gas;
producing draplets (25) of the liquid substance by introducing the liquid substance in a liquid reservoir (12, 35, 95, 125) and presssurising the liquid reservoir to force the liquid substance from the liquid reservoir through a perforated membrane (22,45,97,129) to thereby produce a jet of liquid substance wich is transformed into the draplets of the liquid substance;
supplying the droplets (25) of liquid substance into the high velocity jet of gas;
conveying the droplets (25) of liquid substance within the high velocity jet of gas; and
guiding the high velocity jet of gas toward the surface of the target biological tissue (14, 34, 154) in oder to infect the conveyed droplets (25) into the target biological tissue (14, 34, 154).

2. A method for needleless injecting a liquid substance as defined in claim 1, wherein producing a high velocity jet of gas comprises:
supplying pressurized gas from a gas reservoir (11, 31) to a convergent-divergent (17).

3. A method for needleless injecting a liquid substance as defined in claim 1, wherein producing a high velocity jet of gas comprises:
supplying pressurized gas from a gas reservoir (11, 31) to a convergent (44).

4. A method for needleless injecting a liquid substance as defined in claim 1, wherein the high velocity jet of gas comprises inert gas.

5. A method for needleless injecting a liquid substance as defined in claim 1, wherein pressurizing the liquid reservoir (12, 35, 95, 125) comprises:
supplying pressurized gas from a gas reservoir (11, 31) to the liquid reservoir (12, 35, 95, 125).

6. A method for needleless injecting a liquid substance as defined in claim 1, wherein guiding the high velocity jet of gas comprises:
guiding the flow of the high velocity jet of gas along a face of the perforated membrane (22, 45) on a side of the perforated membrane (22, 45) opposite to the liquid reservoir (12, 35), whereby the jet of the liquid substance (24) and the stream of the droplets (25) resulting from the liquid substance forced through the perforated membrane (22, 45) are supplied within the high velocity jet of gas.

7. A method for needleless injecting a liquid substance as defined in claim 1,comprising producing the high velocity jet of gas prior to producing the droplets (25) to thereby supply the droplets (25) in a steady-state high velocity jet of gas.

8. A needleless syringe (10, 30, 80, 110, 140, 160, 180) for injecting a liquid substance into the skin or other target tissue of a patient (14, 34, 154), comprising: a generator of a high velocity jet of gas comprising a channel for guiding the high velocity jet of gas toward the surface of the skin or other target tissue of a patient;
a generator of draplets of the liquid substance comprising a reservoir (12, 35) of the liquid substance to be injected; an outlet for supplying the draplets (25) of the liquid substance in the high velocity jet of gas;
a perforated membrane (22, 45) interposed between the reservoir of the liquid substance and the channel for guiding the high velocity jet of gas; and a pressurised gas supply connected to the reservoir of the liquid substance to supply pressurised gas to the reservoir and force the liquid substance trough the perforated membrane to thereby produce draplets of the liquid substance supplied in the channel and, therefore, in the high velocity jet of gas;
wherehy, in use, the draplets are conveyed within the velocity jet of gas and thereby injected.

9. A needleless syringe (10, 30, 80) for injecting a liquid substance as recited in claim 8, wherein the generator of high velocity jet of gas comprises:
a convergent-divergent (17, 97) having an inlet; and
a pressurized gas supply connected to the inlet of the convergent-divergent (17, 97) to supply pressurized gas to the convergent-divergent (17, 97) and thereby produce the high velocity jet of gas.

10. A needleless syringe (10) for injecting a liquid substance as recited in claim 9, wherein the pressurized gas supply comprises:
a reservoir (11) of pressurized gas; and
a valve (21) interposed between the reservoir of pressurized gas (11) and the inlet of the convergent-divergent (17) to controllably supply pressurized gas from the reservoir to the inlet of the convergent-divergent (17) and thereby produce the high velocity jet of gas.

11. A needleless syringe (30) for injecting a liquid substance as recited in claim 8, wherein the generator of high velocity jet of gas comprises:
a convergent (44) having an inlet; and
a pressurized gas supply connected to the inlet of the convergent (44) to supply pressurized gas to the convergent (44) and thereby produce the high velocity jet of gas.

12. A needleless syringe (30), for injecting a liquid substance as recited in claim 11, wherein the pressurized gas supply comprises:
a reservoir (31) of pressurized gas; and
a valve (46) interposed between the reservoir of pressurized gas (11) and the inlet of the convergent (44) to controllably supply pressurized gas from the reservoir to the inlet of the convergent (44) and thereby produce the high velocity jet of gas.

13. A needleless syringe (10,30,80, 110, 140, 160, 180) for injecting a liquid substance as recited in claim 8, wherein the high velocity jet of gas comprises inert gas.

14. A needleless syringe (10, 30) for injecting a liquid substance as recited in claim 8, wherein the source of pressure comprises:
a reservoir of pressurized gas (11, 31); and
a valve (21, 46) interposed between the reservoir (11, 31) of pressurized gas and the reservoir (12, 35), of the liquid substance to controllably supply pressurized gas from the gas reservoir (11, 31) of pressurized gas to the reservoir (12, 35) of the liquid substance to thereby force the liquid substance through the perforated membrane (22, 45) and thereby produce droplets (25) supplied in the channel and, therefore, in the high velocity jet of gas.

15. A needless syringe (10, 30) for injecting a liquid substance as recited in claim 8, wherein the perforated membrane (22, 45) comprises a metallic or polymeric membrane provided with at least one micro-orifice (23).

16. A needleless syringe (10) for injecting a liquid substance as recited in claim 8, wherein the generator of high velocity jet of gas comprises:
a convergent-divergent (17) comprising a throat (19) through which the high velocity jet of gas travels;
wherein;
the perforated membrane (22) is interposed between the reservoir (12) of liquid substance and the throat (19) of the convergent-divergent (17).

17. A needleless syringe for injecting a liquid substance as recited in claim 8, wherein the generator of high velocity jet of gas comprises:
a pressurized gas supply;
a convergent-divergent having an inlet; and
an intermediate chamber interposed between the pressurized gas supply and the inlet of the convergent-divergent.

18. A needleless syringe for injecting a liquid substance as recited In claim 17, wherein the generator of high velocity jet of gas comprises:
a first valve interposed between the pressurized gas supply and the intermediate chamber to control supply of pressurized gas from the pressurized gas supply to the intermediate chamber; and
a second valve interposed between the intermediate chamber and the inlet of the convergent-divergent to control supply of pressurized gas from the intermediate chamber to the convergent-divergent.

19. A needleless syringe (30) for injecting a liquid substance as recited in claim 8, wherein the generator of high velocity jet of gas comprises:
a pressurized gas supply;
a convergent (44) having an inlet; and
an intermediate chamber (47) interposed between the pressurized gas supply and the inlet of the convergent (44).

20. A needleless syringe (30) for injecting a liquid substance as recited in claim 19, wherein the generator of high velocity jet of gas comprises:
a first valve (46) interposed between the pressurized gas supply (31) and the intermediate chamber (47) to control supply of pressurized gas from the pressurized gas supply (31) to the intermediate chamber (47); and
a second valve (48) interposed between the intermediate chamber (47) and the inlet of the convergent (44) to control supply of pressurized gas from the intermediate chamber (47) to the convergent (44).

21. A needleless syringe (30) for injecting a liquid substance as recited in claim 8, wherein the generator of droplets further comprises:
an intermediate chamber (42) Interposed between the pressurized gas supply and the reservoir of liquid substance (35).

22. A needleless syringe (30) for injecting a liquid substance as recited in claim 21, wherein the generator of droplets further comprises:
a first valve (41) interposed between the pressurized gas supply and the intermediate chamber (42) to control supply of pressurized gas from the pressurized gas supply to the Intermediate chamber (42); and
a second valve (43) interposed between the intermediate chamber (42) and the reservoir (35) of liquid supply to control supply of pressurized gas from the intermediate chamber (42) to the reservoir of liquid supply.

23. A needleless syringe (30) for injecting a liquid substance as recited in claim 22, wherein the reservoir of liquid substance comprises:
a liquid chamber (32) adjacent to the perforated membrane (45) for containing the liquid substance;
a gas-tight chamber (38); and
a slidable piston (36) interposed between the liquid chamber (32) and the gas-tight chamber (38);
wherein:
the second valve (43) is interposed between the intermediate chamber (42) and the gas-tight chamber (38) to control supply of pressurized gas from the intermediate chamber (42) to the gas-tight chamber (38); and
the supply of pressurized gas to the gas-tight chamber (38) applies a pressure on the slidable piston (36) to compress the liquid substance in the liquid chamber (32) and force the liquid substance through the perforated membrane (45) to thereby produce the droplets (25) of liquid substance supplied in the high velocity jet of gas.

24. A needleless syringe (10) for injecting a liquid substance as recited in claim 10, 12, 14, 18, 20 or 22 wherein each valve is an electronic valve.

25. A needleless syringe (10) for injecting a liquid substance as recited in claim 10,12,14,18,20 or 22 wherein each valve is a mechanical valve comprising elements selected from the group consisting of pistons, springs and plungers.

## Patentansprüche

1. Verfahren zum nadellosen Injizieren einer flüssigen Substanz in ein biologisches Zielgewebe (14, 34, 154), wobei die besagte Substanz keinem lebenden menschlichen oder tierischen Körper verabreicht wird, und das Verfahren aus folgenden Schritten besteht:
- aus der Erzeugung eines Hochgeschwindigkeits-Gasstrahls;
- aus der Erzeugung von Tröpfchen (25) der flüssigen Substanz durch die Einleitung der flüssigen Substanz in einen Flüssigkeitsbehälter (12, 35, 95, 125) und die Druckbeaufschlagung des Flüssigkeitsbehälters, sodass die flüssige Substanz aus dem Flüssigkeitsbehälter durch eine gelochte Membran (22, 45, 97, 129) gepresst wird, um einen Flüssigkeitsstrahl zu erzeugen, der in die Tröpfchen der flüssigen Substanz umgewandelt wird;
- aus dem Einschleusen der Tröpfchen der flüssigen Substanz in den Hochgeschwindigkeits-Gasstrahl;
- aus der Beförderung der Tröpfchen (25) der flüssigen Substanz innerhalb des Hochgeschwindigkeits-Gasstrahls; und
- aus der Führung des Hochgeschwindigkeits-Gasstrahls in Richtung der Oberfläche des biologischen Zielgewebes (14, 34, 154), um die beförderten Tröpfchen (25) in das biologische Zielgewebe (14, 34, 154) zu injizieren.

2. Verfahren zum nadellosen Injizieren einer flüssigen Substanz nach Anspruch 1, bei dem die Erzeugung eines Hochgeschwindigkeits-Gasstrahls aus folgenden Schritten besteht:
- aus der Zufuhr eines unter Druck stehenden Gases aus einem Gasbehälter (11, 31) zu einer konvergent-divergenten Düse (17).

3. Verfahren zum nadellosen Injizieren einer flüssigen Substanz nach Anspruch 1, bei dem die Erzeugung eines Hochgeschwindigkeits-Gasstrahls aus folgenden Schritten besteht:
- aus der Zufuhr eines unter Druck stehenden Gases aus einem Gasbehälter (11, 31) zu einer konvergenten Düse (44).

4. Verfahren zum nadellosen Injizieren einer flüssigen Substanz nach Anspruch 1, bei dem der Hochgeschwindigkeits-Gasstrahl ein Inertgas enthält.

5. Verfahren zum nadellosen Injizieren einer flüssigen Substanz nach Anspruch 1, bei dem die Druckbeaufschlagung des Flüssigkeitsbehälters (12, 35, 95, 125) aus folgenden Schritten besteht:
- aus der Zufuhr eines unter Druck stehenden Gases aus einem Gasbehälter (11, 31) zum Flüssigkeitsbehälter (12, 35, 95, 125).

6. Verfahren zum nadellosen Injizieren einer flüssigen Substanz nach Anspruch 1, bei dem die Führung des Hochgeschwindigkeits-Gasstrahls aus folgenden Schritten besteht:
- aus der Führung des Hochgeschwindigkeits-Gasstrahlflusses entlang einer Fläche der gelochten Membran (22, 45) auf einer Seite der gelochten Membran (22, 45), die dem Flüssigkeitsbehälter (12, 35) gegenüber liegt, wodurch der Strahl der flüssigen Substanz (24) und der Tröpfchenstrom (25), der aus der flüssigen Substanz entsteht, die durch die gelochte Membran (22, 45) gepresst wird, in den Hochgeschwindigkeits-Gasstrahl eingeschleust werden.

7. Verfahren zum nadellosen Injizieren einer flüssigen Substanz nach Anspruch 1, einschließlich der Erzeugung des Hochgeschwindigkeits-Gasstrahls vor der Erzeugung der Tröpfchen (25), um dabei die Tröpfchen (25) in einen stabilisierten Hochgeschwindigkeits-Gasstrahl einzuleiten.

8. Nadellose Spritze (10, 30, 80, 110, 140, 160, 180) zum Injizieren einer flüssigen Substanz in die Haut oder in jedes andere Zielgewebe eines Patienten (14, 34, 154), mit:
- einem Generator eines Hochgeschwindigkeits-Gasstrahls mit einem Kanal zur Führung des Hochgeschwindigkeits-Gasstrahls in Richtung der Oberfläche der Haut oder jedes anderen Zielgewebes eines Patienten;
- einem Tröpfchengenerator der flüssigen Substanz, bestehend aus: einem Behälter (12, 35) mit der zu injizierenden flüssigen Substanz; einem Ausgang zum Einschleusen der Tröpfchen (25) der flüssigen Substanz in den Hochgeschwindigkeits-Gasstrahl; einer gelochten Membran (22, 45), die zwischen dem Behälter der flüssigen Substanz und dem Kanal zur Führung des Hochgeschwindigkeits-Gasstrahls eingeschoben ist; und einer Druckgaszuführung, die mit dem Flüssigkeitsbehälter verbunden ist, um ein unter Druck stehendes Gas in den Behälter zu liefern und die flüssige Substanz durch die gelochte Membran zu pressen, um so die Tröpfchen der flüssigen Substanz zu erzeugen, die in den Kanal, und somit auch in den Hochgeschwindigkeits-Gasstrahl eingeschleust werden;
wodurch die Tröpfchen bei der Verwendung in den Hochgeschwindigkeits-Gasstrahl geführt, und somit auch injiziert werden.

9. Nadellose Spritze (10, 30, 80) zum Injizieren einer flüssigen Substanz nach Anspruch 8, bei der der Generator des Hochgeschwindigkeits-Gasstrahls folgendes enthält:
- eine konvergent-divergente Düse (17, 97) mit einem Einlass; und
- eine Druckgaszuführung, die mit dem Einlass der konvergent-divergenten Düse (17, 97) verbunden ist, um der konvergent-divergenten Düse (17, 97) ein unter Druck stehendes Gas zu liefern, und somit den Hochgeschwindigkeits-Gasstrahl zu erzeugen.

10. Nadellose Spritze (10) zum Injizieren einer flüssigen Substanz nach Anspruch 9, bei der Druckgaszuführung folgendes enthält:
- einen Behälter (11) mit unter Druck stehendem Gas; und
- ein Ventil (21) zwischen dem Druckgasbehälter (11) und dem Einlass der konvergent-divergenten Düse (17) zur gesteuerten Zuführung des unter Druck stehenden Gases aus dem Behälter bis zum Einlass der konvergent-divergenten Düse (17) und zur Erzeugung des Hochgeschwindigkeits-Gasstrahls.

11. Nadellose Spritze (30) zum Injizieren einer flüssigen Substanz nach Anspruch 8, bei der der Generator des Hochgeschwindigkeits-Gasstrahls folgendes enthält:
- eine konvergente Düse (44) mit einem Einlass; und
- eine Druckgaszuführung, die mit dem Einlass der konvergenten Düse (44) verbunden ist, um der konvergenten Düse (44) ein unter Druck stehendes Gas zu liefern, und somit den Hochgeschwindigkeits-Gasstrahl zu erzeugen.

12. Nadellose Spritze (30) zum Injizieren einer flüssigen Substanz nach 11, bei der die Druckgaszuführung folgendes enthält:
- einen Behälter (31) mit unter Druck stehendem Gas; und
- ein Ventil (46) zwischen dem Druckgasbehälter (11) und dem Einlass der konvergenten Düse (44) zur gesteuerten Zuführung des unter Druck stehenden Gases aus dem Behälter bis zum Einlass der konvergenten Düse (44) und zur Erzeugung des Hochgeschwindigkeits-Gasstrahls.

13. Nadellose Spritze (10, 30, 80, 110, 140, 160, 180) zum Injizieren einer flüssigen Substanz nach Anspruch 8, bei der der Hochgeschwindigkeits-Gasstrahl ein Inertgas enthält.

14. Nadellose Spritze (10, 30) zum Injizieren einer flüssigen Substanz nach Anspruch 8, bei der die Druckquelle folgendes enthält:
- einen Druckgasbehälter (11, 31); und
- ein Ventil (21, 46) zwischen dem Druckgasbehälter (11, 31) und dem Behälter (12, 35) der flüssigen Substanz zur gesteuerten Zuführung des unter Druck stehenden Gases vom Druckgasbehälter (11, 31) bis zum Behälter (12, 35) der flüssigen Substanz, um die flüssige Substanz durch die gelochte Membran (22, 35) zu pressen, und somit die Tröpfchen (25) herzustellen, die in den Kanal, und somit auch in den Hochgeschwindigkeits-Gasstrahl eingeschleust werden.

15. Nadellose Spritze (10, 30) zum Injizieren einer flüssigen Substanz nach Anspruch 8, bei der die gelochte Membran (22, 45) eine Metall- oder Polymermembran enthält, die zumindest eine Mikro-Öffnung (23) aufweist.

16. Nadellose Spritze (10) zum Injizieren einer flüssigen Substanz nach Anspruch 8, bei der der Generator des Hochgeschwindigkeits-Gasstrahls folgendes enthält:
- eine konvergent-divergente Düse (17) mit einer Rinne (19), durch die der Hochgeschwindigkeits-Gasstrahl verläuft;
worin:
- die gelochte Membran (22) zwischen dem Behälter (12) der flüssigen Substanz und der Rinne (19) der konvergent- divergenten Düse (17) eingeschoben ist.

17. Nadellose Spritze zum Injizieren einer flüssigen Substanz nach Anspruch 8, bei der der Generator des Hochgeschwindigkeits-Gasstrahls folgendes enthält:
- eine Druckgaszuführung;
- eine konvergent-divergente Düse mit einem Einlass; und
- eine Zwischenkammer zwischen der Druckgaszuführung und dem Einlass der konvergent-divergenten Düse.

18. Nadellose Spritze zum Injizieren einer flüssigen Substanz nach Anspruch 17, bei der der Generator des Hochgeschwindigkeits-Gasstrahls folgendes enthält:
- ein erstes Ventil zwischen der Druckgaszuführung und der Zwischenkammer zur Steuerung der Druckgaszufuhr von der Druckgaszuführung bis zur Zwischenkammer; und
- ein zweites Ventil zwischen der Zwischenkammer und dem Einlass der konvergent-divergenten Düse zur Steuerung der Druckgaszufuhr von der Zwischenkammer bis zur konvergent-divergenten Düse.

19. Nadellose Spritze (30) zum Injizieren einer flüssigen Substanz nach Anspruch 8, bei der der Generator des Hochgeschwindigkeits-Gasstrahls folgendes enthält:
- eine Druckgaszuführung;
- eine konvergente Düse (14) mit einem Einlass; und
- eine Zwischenkammer (47) zwischen der Druckgaszuführung und dem Einlass der konvergenten Düse (44).

20. Nadellose Spritze (30) zum Injizieren einer flüssigen Substanz nach Anspruch 19, bei der der Generator des Hochgeschwindigkeits-Gasstrahls folgendes enthält:
- ein erstes Ventil (46) zwischen der Druckgaszuführung (31) und der Zwischenkammer (47) zur Steuerung der Druckgaszufuhr von der Druckgaszuführung (31) bis zur Zwischenkammer (47); und
- ein zweites Ventil (48) zwischen der Zwischenkammer (47) und dem Einlass der konvergenten Düse (44) zur Steuerung der Druckgaszufuhr von der Zwischenkammer (47) bis zur konvergenten Düse (44).

21. Nadellose Spritze (30) zum Injizieren einer flüssigen Substanz nach Anspruch 8, bei der der Tröpfchengenerator darüber hinaus folgendes enthält:
- eine Zwischenkammer (42) zwischen der Druckgaszuführung und dem Behälter der flüssigen Substanz (35).

22. Nadellose Spritze (30) zum Injizieren einer flüssigen Substanz nach Anspruch 21, bei der der Tröpfchengenerator darüber hinaus folgendes enthält:
- ein erstes Ventil (41) zwischen der Druckgaszuführung und der Zwischenkammer (42) zur Steuerung der Druckgaszufuhr von der Druckgaszuführung bis zur Zwischenkammer (42); und
- ein zweites Ventil (43) zwischen der Zwischenkammer (42) und dem Behälter (35) für die Flüssigkeitszuführung zur Steuerung der der Druckgaszufuhr von der Zwischenkammer (42) bis zum Behälter für die Flüssigkeitszuführung.

23. Nadellose Spritze (30) zum Injizieren einer flüssigen Substanz nach Anspruch 22, bei der der Flüssigkeitsbehälter folgendes enthält:
- eine Flüssigkeitskammer (32) neben der gelochten Membran (45) zur Aufnahme der flüssigen Substanz;
- eine gasdichte Kammer (38); und
- einen Gleitkolben (36) zwischen der Flüssigkeitskammer (32) und der gasdichten Kammer (38);
worin:
- das zweite Ventil (43) zwischen der Zwischenkammer (42) und der gasdichten Kammer (38) eingefügt ist, zur Steuerung der Druckgaszuführung von der Zwischenkammer (42) bis zur gasdichten Kammer (38); und
- die Druckgaszuführung zur gasdichten Kammer (38) einen Druck auf den Gleitkolben (36) ausübt, um die flüssige Substanz in der Flüssigkeitskammer (32) zu komprimieren und die flüssige Substanz durch die gelochte Membran (45) zu pressen, um so die Tröpfchen (25) der flüssigen Substanz zu bilden, die in den Hochgeschwindigkeits-Gasstrahl eingeschleust werden.

24. Nadellose Spritze (10) zum Injizieren einer flüssigen Substanz nach den Ansprüchen 10, 12, 14, 18, 20 oder 22, bei der jedes Ventil ein elektronisches Ventil ist.

25. Nadellose Spritze (10) zum Injizieren einer flüssigen Substanz nach den Ansprüchen 10, 12, 14, 18, 20 oder 22, bei der jedes Ventil ein mechanisches Ventil ist, mit Elementen, die aus der Gruppe bestehend aus den Kolben, Federn und Plungern ausgewählt werden.

## Revendications

1. Procédé d'injection sans aiguille d'une substance liquide dans un tissu biologique cible (14, 34, 154), moyennant quoi ladite substance n'est pas dispensée au corps vivant humain ou animal, le procédé comprenant :
- la production d'un jet de gaz à grande vitesse ;
- la production de gouttelettes (25) de la substance liquide par introduction de la substance liquide dans un réservoir à liquide (12, 35, 45, 125) et mise sous pression du réservoir à liquide de manière à pousser la substance liquide depuis le réservoir à liquide à travers une membrane perforée (22, 45, 97, 129) pour produire ainsi un jet de substance liquide qui est transformé en les gouttelettes de la substance liquide ;
- la fourniture des gouttelettes de substance liquide dans le jet de gaz à grande vitesse ;
- l'acheminement des gouttelettes (25) de substance liquide dans le jet de gaz à grande vitesse ; et
- le guidage du jet de gaz à grande vitesse sur la surface du tissu biologique cible (14, 34, 154) de manière à injecter les gouttelettes acheminées (25) dans le tissu biologique cible (14, 34, 154).

2. Procédé d'injection sans aiguille d'une substance liquide tel que défini dans la revendication 1, dans lequel la production d'un jet de gaz à grande vitesse comprend :
- la fourniture de gaz sous pression depuis un réservoir à gaz (11, 31) à une tuyère convergente - divergente (17).

3. Procédé d'injection sans aiguille d'une substance liquide tel que défini dans la revendication 1, dans lequel la production d'un jet de gaz à grande vitesse comprend :
- la fourniture de gaz sous pression depuis un réservoir à gaz (11, 31) à un convergent (44).

4. Procédé d'injection sans aiguille d'une substance liquide tel que défini dans la revendication 1, dans lequel le jet de gaz à grande vitesse comprend un gaz inerte.

5. Procédé d'injection sans aiguille d'une substance liquide tel que défini dans la revendication 1, dans lequel la mise sous pression du réservoir à liquide (12, 35, 95, 125) comprend :
- la fourniture de gaz sous pression depuis un réservoir à gaz (11, 31) au réservoir à liquide (12, 35, 95, 125).

6. Procédé d'injection sans aiguille d'une substance liquide tel que défini dans la revendication 1, dans lequel le guidage du jet de gaz à grande vitesse comprend :
- le guidage de l'écoulement du jet de gaz à grande vitesse sur une face de la membrane perforée (22, 45) d'un côté de la membrane perforée (22, 45) opposé au réservoir à liquide (12, 35), moyennant quoi le jet de la substance liquide (24) et le courant des gouttelettes (25) résultant de la substance liquide poussée à travers la membrane perforée (22, 45) sont fournis dans le jet de gaz à grande vitesse.

7. Procédé d'injection sans aiguille d'une substance liquide tel que défini dans la revendication 1, comprenant la production du jet de gaz à grande vitesse préalablement à la production des gouttelettes (25) pour fournir ainsi les gouttelettes (25) dans un jet de gaz à grande vitesse stabilisé.

8. Seringue sans aiguille (10, 30, 80, 110, 140, 160, 180) pour injecter une substance liquide dans la peau ou tout autre tissu cible d'un patient (14, 34, 154), comprenant :
- un générateur d'un jet de gaz à grande vitesse comprenant un canal pour guider le jet de gaz à grande vitesse sur la surface de la peau ou de tout autre tissu cible d'un patient ;
- un générateur de gouttelettes de la substance liquide comprenant : un réservoir (12, 35) de la substance liquide à injecter ; une sortie pour fournir les gouttelettes (25) de la substance liquide dans le jet de gaz à grande vitesse ; une membrane perforée (22, 45) interposée entre le réservoir de la substance liquide et le canal pour guider le jet de gaz à grande vitesse ; et une alimentation en gaz sous pression reliée au réservoir de la substance liquide pour fournir un gaz sous pression au réservoir et pousser la substance liquide à travers la membrane perforée pour produire ainsi des gouttelettes de la substance liquide fournies dans le canal et, par conséquent, dans le jet de gaz à grande vitesse ;
moyennant quoi, lors de l'utilisation, les gouttelettes sont acheminées dans le jet de gaz à grande vitesse et ainsi injectées.

9. Seringue sans aiguille (10, 30, 80) pour injecter une substance liquide telle que décrite dans la revendication 8, dans laquelle le générateur de jet de gaz à grande vitesse comprend :
- une tuyère convergente - divergente (17, 97) ayant une admission ; et
- une alimentation en gaz sous pression reliée à l'admission de la tuyère convergente - divergente (17, 97) pour fournir un gaz sous pression à la tuyère convergente - divergente (17, 97) et produire ainsi le jet de gaz à grande vitesse.

10. Seringue sans aiguille (10) pour injecter une substance liquide telle que décrite dans la revendication 9, dans laquelle l'alimentation en gaz sous pression comprend :
- un réservoir (11) de gaz sous pression ; et
- une valve (21) interposée entre le réservoir de gaz sous pression (11) et l'admission de la tuyère convergente - divergente (17) pour fournir de manière contrôlée le gaz sous pression depuis le réservoir jusqu'à l'admission de la tuyère convergente - divergente (17) et produire ainsi le jet de gaz à grande vitesse.

11. Seringue sans aiguille (30) pour injecter une substance liquide telle que décrite dans la revendication 8, dans laquelle le générateur de jet de gaz à grande vitesse comprend :
- un convergent (44) ayant une admission ; et
- une alimentation en gaz sous pression reliée à l'admission du convergent (44) pour fournir le gaz sous pression au convergent (44) et produire ainsi le jet de gaz à grande vitesse.

12. Seringue sans aiguille (30) pour injecter une substance liquide telle que décrite dans la revendication 11, dans laquelle l'alimentation en gaz sous pression comprend :
- un réservoir (31) de gaz sous pression ; et
- une valve (46) interposée entre le réservoir de gaz sous pression (11) et l'admission du convergent (44) pour fournir de manière contrôlée le gaz sous pression depuis le réservoir jusqu'à l'admission du convergent (44) et produire ainsi le jet de gaz à grande vitesse.

13. Seringue sans aiguille (10, 30, 80, 110, 140, 160, 180) pour injecter une substance liquide telle que décrite dans la revendication 8, dans laquelle le jet de gaz à grande vitesse comprend un gaz inerte.

14. Seringue sans aiguille (10, 30)- pour injecter une substance liquide telle que décrite dans la revendication 8, dans laquelle la source de pression comprend :
- un réservoir de gaz sous pression (11, 31) ; et
- une valve (21, 46) interposée entre le réservoir (11, 31) de gaz sous pression et le réservoir (12, 35) de la substance liquide pour fournir de manière contrôlée le gaz sous pression depuis le réservoir à gaz (11, 31) de gaz sous pression jusqu'au réservoir (12, 35) de la substance liquide pour pousser ainsi la substance liquide à travers la membrane perforée (22, 35) et produire ainsi des gouttelettes (25) fournies dans le canal et, par conséquent, dans le jet de gaz à grande vitesse.

15. Seringue sans aiguille (10, 30) pour injecter une substance liquide telle que décrite dans la revendication 8, dans laquelle la membrane perforée (22, 45) comprend une membrane métallique ou polymère munie d'au moins un micro-orifice (23).

16. Seringue sans aiguille (10) pour injecter une substance liquide telle que décrite la revendication 8, dans laquelle le générateur de jet de gaz à grande vitesse comprend :
- une tuyère convergente - divergente (17) comprenant une gorge (19) au travers de laquelle le jet de gaz à grande vitesse évolue ;
dans laquelle :
- la membrane perforée (22) est interposée entre le réservoir (12) de substance liquide et la gorge (19) de la tuyère convergente - divergente (17).

17. Seringue sans aiguille pour injecter une substance liquide telle que décrite dans la revendication 8, dans laquelle le générateur de jet de gaz à grande vitesse comprend :
- une alimentation en gaz sous pression ;
- une tuyère convergente - divergente ayant une admission ; et
- une chambre intermédiaire interposée entre l'alimentation en gaz sous pression et l'admission de la tuyère convergente - divergente.

18. Seringue sans aiguille pour injecter une substance liquide telle que décrite dans la revendication 17, dans laquelle le générateur de jet de gaz à grande vitesse comprend :
- une première valve interposée entre l'alimentation en gaz sous pression et la chambre intermédiaire pour contrôler la fourniture de gaz sous pression depuis l'alimentation en gaz sous pression jusqu'à la chambre intermédiaire ; et
- une seconde valve interposée entre la chambre intermédiaire et l'admission de la tuyère convergente - divergente pour contrôler la fourniture de gaz sous pression depuis la chambre intermédiaire jusqu'à la tuyère convergente - divergente.

19. Seringue sans aiguille (30) pour injecter une substance liquide telle que décrite dans la revendication 8, dans laquelle le générateur de jet de gaz à grande vitesse comprend :
- une alimentation en gaz sous pression ;
- un convergent (14) ayant une admission ; et
- une chambre intermédiaire (47) interposée entre l'alimentation en gaz sous pression et l'admission du convergent (44).

20. Seringue sans aiguille (30) pour injecter une substance liquide telle que décrite dans la revendication 19, dans laquelle le générateur de jet de gaz à grande vitesse comprend :
- une première valve (46) interposée entre l'alimentation en gaz sous pression (31) et la chambre intermédiaire (47) pour contrôler la fourniture de gaz sous pression depuis l'alimentation en gaz sous pression (31) jusqu'à la chambre intermédiaire (47) ; et
- une seconde valve (48) interposée entre la chambre intermédiaire (47) et l'admission du convergent (44) pour contrôler la fourniture de gaz sous pression depuis la chambre intermédiaire (47) jusqu'au convergent (44).

21. Seringue sans aiguille (30) pour injecter une substance liquide telle que décrite dans la revendication 8, dans laquelle le générateur de gouttelettes comprend en outre :
- une chambre intermédiaire (42) interposée entre l'alimentation en gaz sous pression et le réservoir de substance liquide (35).

22. Seringue sans aiguille (30) pour injecter une substance liquide telle que décrite dans la revendication 21, dans laquelle le générateur de gouttelettes comprend en outre :
- une première valve (41) interposée entre l'alimentation en gaz sous pression et la chambre intermédiaire (42) pour contrôler la fourniture de gaz sous pression depuis l'alimentation en gaz sous pression jusqu'à la chambre intermédiaire (42) ; et
- une seconde valve (43) interposée entre la chambre intermédiaire (42) et le réservoir (35) de fourniture de liquide pour contrôler la fourniture de gaz sous pression depuis la chambre intermédiaire (42) jusqu'au réservoir de fourniture de liquide.

23. Seringue sans aiguille (30) pour injecter une substance liquide telle que décrite dans la revendication 22, dans laquelle le réservoir de substance liquide comprend :
- une chambre à liquide (32) adjacente à la membrane perforée (45) pour contenir la substance liquide ;
- une chambre étanche aux gaz (38) ; et
- un piston coulissant (36) interposé entre la chambre à liquide (32) et la chambre étanche aux gaz (38) ;
dans laquelle :
- la seconde valve (43) est interposée entre la chambre intermédiaire (42) et la chambre étanche aux gaz (38) pour contrôler la fourniture de gaz sous pression depuis la chambre intermédiaire (42) jusqu'à la chambre étanche aux gaz (38) ; et
- la fourniture de gaz sous pression à la chambre étanche aux gaz (38) applique une pression sur le piston coulissant (36) pour comprimer la substance liquide dans la chambre à liquide (32) et pousser la substance liquide à travers la membrane perforée (45) pour produire ainsi les gouttelettes (25) de substance liquide fournies dans le jet de gaz à grande vitesse.

24. Seringue sans aiguille (10) pour injecter une substance liquide telle que décrite dans les revendications 10, 12, 14, 18, 20 ou 22, dans laquelle chaque valve est une valve électronique.

25. Seringue sans aiguille (10) pour injecter une substance liquide telle que décrite dans les revendications 10, 12, 14, 18, 20 ou 22, dans laquelle chaque valve est une valve mécanique comprenant des éléments choisis dans le groupe constitué par les pistons, les ressorts et les pistons plongeurs.
